# EUROPEAN PATENT SPECIFICATION

(11) **EP 2 294 144 B1**
(45) Date of publication and mention of the grant of the patent: **05.03.2014**
(21) Application number: 09746886.2
(22) Date of filing: 18.05.2009
(51) Int. Cl.: C09D 5/16, C09D 7/12

(54) **ANTIFOULING COMPOUNDS AND USE THEREOF**
ANTIFOULING-VERBINDUNGEN UND IHRE VERWENDUNG
COMPOSÉS ANTISALISSURES ET LEUR UTILISATION

(30) Priority: 16.05.2008 US 53729 P
(43) Date of publication of application: 16.03.2011
(73) Proprietor: National University of Singapore, Singapore 119077 (SG); Agency for Science, Technology And Research, Singapore 138632 (SG); Maritime and Port Authority of Singapore, Singapore 119963 (SG)
(72) Inventor: TEO, Lay Ming, Serena, Singapore 119227 (SG); RITTSCHOF, Daniel, Moorehead City North Carolina 28557 (US); JAMESON, Felicity, Symonston ACT 2609 (AU); CHAI, Christina, Singapore 627833 (SG); CHEN, Chia Lung, Singapore (SG); LEE, Siew Chen, Serina, Singapore 119227 (SG)
(74) Representative: Webster, Jeremy Mark
(86) International application number: PCT/SG2009/000175
(87) International publication number: WO 2009/139729

(56) References cited:
- EP-A1- 0 835 605
- EP-A1- 1 245 558
- WO-A1-99/05912
- US-A- 3 378 563
- US-A- 6 030 971

## Description

### FIELD OF THE INVENTION

The present invention is concerned with small molecules that exhibit antifouling and/or antibacterial activity and their use in the control of bacterial films and organism growth in the marine environment.

### BACKGROUND

There is a continuing and growing need to find alternatives to conventional marine antibacterial and antifouling agents.

In October 2001, the International Maritime Organisation (IMO) adopted the International Convention of the Control of Harmful Antifouling Systems (AFS Convention) which prohibited the use of organotin species as antifouling agents in marine coatings (Harino, 2007). Following this, since 2003, tin-based paints have been withdrawn by most of the major paint companies.

Subsequent generations of marine coatings have contained high levels of copper and/or booster biocides, which are often highly toxic pesticides developed for agricultural purposes. These additives have also been shown to build up in the environment with detrimental side effects and are now regulated in many parts of Europe (Bellas, 2006). Indeed, environmental studies of several new antifouling booster biocides indicate that these complex molecules may be less degradable than expected as they are detectable in harbour waters (Voulvoulis, 2006).

Current commercial marine coatings can be divided into two classes: antifouling and foul-release. Antifouling coatings use broad-spectrum biocides which kill fo5ulers by oxidation or, more usually, exposure to toxic metal ions. Foul-release coatings are mainly silicon based polymers that are easy to clean, however the best of these usually also contain additives and catalysts that kill organisms. Legislation and agreements, based on the recognition of the environmentally unacceptable consequences of toxic antifouling agents such as tributyl tin in coatings, have prompted interest to develop new less environmentally pernicious coatings.

An approach reported by Teo et al (an inventor of the present application) in US 11/265,833, is the use of pharmaceuticals as antifouling agents. It has been demonstrated that pharmaceuticals may disrupt the metamorphosis of fouling organisms. Commercially available pharmaceuticals, with their known synthesis, chemical properties and primary mechanism of action in vertebrates and in humans, were screened as potential sources of antifouling agents. Whilst eight pharmaceuticals with promising bioactivity were reported, there remains the problem that these pharmaceuticals may accumulate in the marine environment. Furthermore, some of these pharmaceuticals suffer from delivery problems because of poor solubility in sea water.

Thus, there remains a need for marine antibacterial and antifouling agents which are not only sufficiently bioactive but also deliverable in seawater and degradable so as to avoid accumulation.

US 6,030,971 relates to a method of inhibiting bacterial adhesion to a submergible surface in aqueous systems by using effective amount of amides, which is below the toxic threshold levels.

### SUMMARY OF THE INVENTION

### Compounds

The present invention pertains generally to a class of compounds referred to herein as "antifouling amide compounds", which compounds have the following general formula (I) 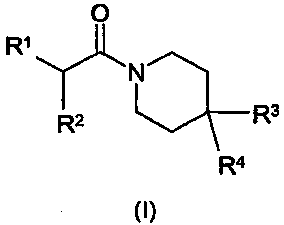 wherein
R¹ and R² are independently selected from optionally substituted aryl, optionally substituted C₃ to C₁₂ alkyl; and
R³ and R⁴ are independently selected from hydroxy, optionally substituted C₁ to C₆ alkyl, optionally substituted phenyl and H.

The present invention pertains to such antifouling amide compounds, which exhibit biocidal or biostatic properties. Therefore, the antifouling amide compounds may also be referred to as "biocidal compounds" or "biostatic compounds".

In a first aspect the present invention provides a use of an antifouling amide compound of formula (I) in a method of preventing or reducing fouling.

The present inventors have found that an amide functionality wherein the nitrogen of the amide is part of a piperidine ring can provide antifouling activity (including one or both of antibacterial and anti-settlement activity) and preferably also levels of degradation which make the compounds attractive alternatives to known antifouling compounds. In particular, the observed activity is surprising because the pharmaceutical loperamide studied by the present inventors does not include the amide-piperidine functionality referred to above.

Suitably R¹ and R² are independently selected from aryl and C₃ to C₁₀ alkyl; preferably from aryl, C₃ to C₈ alkyl, more preferably from aryl, and C₃ to C₆ alkyl. In each case, the aryl or alkyl may be substituted and this applies to the subsequent discussion of these groups herein.

The aryl, if present, is preferably C₅ to C₃₀ aryl, more preferably C₅ to C₂₀ aryl, more preferably C₅ to C₁₅ aryl, more preferably C₅ to C₁₂ aryl, more preferably C₅ to C₁₀ aryl, more preferably C₅ to C₈ aryl, more preferably C₅ to C₇ aryl and most preferably C₆ aryl, and is optionally substituted.

They aryl may be carboaryl or heteroaryl. Carboaryl is preferred.

A particularly preferred aryl is phenyl.

Suitably the aryl is unsubstituted.

Preferably at least one of R¹ and R² is C₃ to C₁₂ alkyl, more preferably C₃ to C₁₀ alkyl, more preferably C₃ to C₈ alkyl and most preferably C₃ to C₆ alkyl. The present inventors have found that the presence of an alkyl chain on the alpha carbon can provide antifouling activity. In some preferred embodiments, the alkyl is unsaturated alkyl. In particular, preferably at least one of R¹ and R² is unsaturated C₃ to C₁₂ alkyl, preferably unsaturated C₃ to C₁₀ alkyl, preferably unsaturated C₃ to C₈ alkyl and most preferably unsaturated C₃ to C₆ alkyl. Thus, preferably at least one of R¹ and R² is C₃ to C₁₂ alkenyl, more preferably C₃ to C₁₀ alkenyl, more preferably C₃ to C₈ alkenyl and most preferably C₃ to C₈ alkenyl. Indeed, the present inventors have found that the addition of unsaturation can provide activity comparable to the saturated alkyl.

In such embodiments, preferably there is one double bond in the alkenyl, for example one double bond in C₃ to C₁₀ alkenyl, or one double bond in C₃ to C₆ alkenyl. Suitably the double bond is at the end of the alkenyl group, i.e. between the Cₙ and Cₙ₋₁ carbons. In other embodiments, two double bonds are present. C₅ and C₆ alkenyls are preferred. A particularly preferred alkenyl is C₆ alkenyl, most preferably 5-hexenyl (-CH₂-(CH₂)₃-CH=CH₂). A further preferred alkenyl is -CH=CH-CH=CH-CH₃. Nevertheless, saturated alkyl groups are preferred.

Preferably at least one of R¹ and R² is C₃ to C₅ alkyl. The present inventors have found that alkyl groups on the alpha carbon having between 3 and 5 carbon atoms are particularly useful in providing antifouling activity whilst also exhibiting desirable solubility in sea water and degradability.

Preferably at least one of R¹ and R² is C₄ alkyl, more preferably n-butyl. The studies conducted by the present inventors have shown that a C₄ alkyl group, and in particular n-butyl, on the alpha carbon can provide surprisingly high levels of antifouling activity and is degraded at an appropriate rate.

Suitably one of R¹ and R² is aryl (preferably C₆ to C₁₅ aryl, more preferably phenyl) and the other is C₃ to C₁₂ alkyl (preferably C₃ to C₆ alkyl, more preferably n-butyl). The tests conducted by the present inventors demonstrate that desirable levels of antifouling activity are possible with this substitution pattern.

R¹ and R² are not H. In this connection, the present inventors have found that di-substitution at the alpha carbon is desirable.

Preferably R¹ and R² are the same. Most preferably R¹ and R² are both n-butyl. The antifouling studies conducted by the present inventors shows that di n-butyl substitution at the alpha carbon provides excellent antifouling activity. In particular, a broad range of antibacterial activity has been observed as well as anti-settlement activity. Furthermore, high therapeutic ratio (TR) values are achieved, indicating that such compounds provide a useful antifouling effect whilst having a comparatively low level of toxicity.

Preferably one or both of R¹ and R² are unsubstituted. Most preferably, both are unsubstituted. Thus, whilst substitution of R¹ and R² is possible, the present inventors believe that the best overall performance in terms of antifouling effect and degradability is achieved without substitution. In particular, the absence of halogen substituents has been found to be particularly desirable, particularly with reference to the degradability of the compounds. Similarly, the absence of hydroxy substituents is also preferred.

Preferably one or both of R¹ and R² are saturated. Most preferably, both are saturated.

In other preferred embodiments, R¹ and R² are both phenyl. Compounds having this substitution pattern have been found to exhibit antibacterial activity across a broad range of bacteria, as well as anti-settlement activity. Comparatively low levels of toxicity are also observed for this preferred arrangement.

In certain preferred embodiments, one of R³ and R⁴ is hydroxyl and the other is H. In even more preferred embodiments both of R³ and R⁴ are H.

In other preferred embodiments, one of R³ and R⁴ is substituted C₁ - C₆ alkyl. Particularly preferred is hydroxy-C₁ - C₆ alkyl, preferably -CH₂CH₂OH. Suitably the other one of R³ and R⁴ is H.

Preferably one or both of R³ and R⁴ are unsubstituted. Most preferably, both are unsubstituted. Thus, whilst substitution of R¹ and R² is possible, the present inventors believe that the best overall performance in terms of antifouling effect and degradability is achieved without substitution. In particular, the absence of halogen substituents has been found to be particularly desirable, particularly with reference to the degradability of the compounds.

A particularly preferred combination of substituents is as follows:
(i) at least one of R¹ and R² is n-butyl
(ii) both of R³ and R⁴ are H

In such preferred combinations where only one of R¹ and R² is n-butyl, it is preferred that the other one is aryl, preferably C₅ to C₁₅ aryl, more preferably phenyl.

In the most preferred compounds, none of the substituents are phenyl and preferably none of the substituents are aryl.

Suitably the compound is selected from compounds 12.2, 12.1, 12.7, 12.4, 12.5, 12.6, 12.8, 12.9, 12.10, 12.11, 12.12, 4.1, 9.3, 4.5 and 4.3. Preferably the compound is selected from compounds 12.2, 12.1, 12.7, 12.4, 12.5, 12.8, 12.9, 12.10, 12.11, 12.12, 4.1, 9.3, 4.5, 4.3, and 10.7. More preferably the compound is selected from compounds 12.2, 12.1, 12.7, 12.4, 12.8, 12.9, 12.10, 12.11, 12.12, 4.1, 9.3, 4.5 and 4.3. Particularly preferred are compounds 12.2, 12.1, 12.7, 12.4, 12.8, 12.9 and 4.1. Especially preferred are compounds 12.1, 12.2, 12.4,12.7 and 12.8, more preferably compounds 12.1 and 12.2 and most preferably compound 12.2. Also especially preferred is compound 4.1.

In a further aspect, the present invention provides a use of a compound selected from 4.1, 9.3, 9.2, 4.5, 4.3, 12.1, 12.2, 12.4, 12.7, 12.8, 12.9, 12.10, 12.11, 12.12 and 11.2 in a method of preventing or reducing fouling. Particularly preferred are compounds 12.3 and 11.2, especially compound 12.3.

In a further aspect, the present invention provides a use of a novel compound 9.2 in a method of preventing or reducing fouling.

In a further aspect, the present invention provides a method of preventing or reducing fouling of a substrate, wherein the method comprises the step of applying an antifouling amide compound as described herein to the substrate.

Suitably the antifouling amide compound is applied at in an amount and at a concentration effective to prevent or reduce fouling. Preferably the antifouling amide compound is provided at a standard concentration.

In a further aspect, the present invention provides an antifouling composition comprising an antifouling amide compound as described herein.

In a further aspect, the present invention provides a coating composition comprising an antifouling amide compound. Suitably the coating composition comprises conventional additives, for example a binder. Suitably the coating composition is a paint composition. For example, the composition can include an acrylate resin. Suitably the coating composition is a self-polishing paint, preferably an acrylic self polishing paint, or a silicone coating.

In a further aspect, the present invention provides a coating comprising an antifouling amide compound as described herein.

In a further aspect, the present invention provides a substrate having a coating applied thereto, wherein the coating comprises an antifouling amide compound as described herein. For example, the substrate may be a vessel, for example a boat.

In a further aspect, the present invention provides a bacteriostatic composition comprising an antifouling amide compound as described herein.

In a further aspect, the present invention provides a bacteriocidal composition comprising an antifouling amide compound as described herein.

In a further aspect, the present invention provides a biocidal composition comprising an antifouling amide compound as described herein.

In a further aspect, the present invention provides a biostatic composition comprising an antifouling amide compound as described herein.

In a further aspect, the present invention provides an antifoulant composition comprising an antifouling amide compound as described herein.

Any one or more of the optional and preferred features of any of the aspects may apply, singly or in combination, to any one of the other aspects.

### DETAILED DESCRIPTION OF THE INVENTION

### Chemical Terms

The term "saturated," as used herein, pertains to compounds and/or groups which do not have any carbon-carbon double bonds or carbon-carbon triple bonds.

The term "unsaturated," as used herein, pertains to compounds and/or groups which have at least one carbon-carbon double bond or carbon-carbon triple bond. Compounds and/or groups may be partially unsaturated or fully unsaturated.

The term "carbo," "carbyl," "hydrocarbo," and "hydrocarbyl," as used herein, pertain to compounds and/or groups which have only carbon and hydrogen atoms.

The term "hetero," as used herein, pertains to compounds and/or groups which have at least one heteroatom, for example, multivalent heteroatoms (which are also suitable as ring heteroatoms) such as boron, silicon, nitrogen, phosphorus, oxygen, sulfur, and selenium (more commonly nitrogen, oxygen, and sulfur) and monovalent heteroatoms, such as fluorine, chlorine, bromine, and iodine.

The phrase "optionally substituted," as used herein, pertains to a parent group which may be unsubstituted or which may be substituted.

Unless otherwise specified, the term "substituted," as used herein, pertains to a parent group which bears one or more substitutents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

Alkyl: The term "alkyl," as used herein, pertains to a monovalent moiety obtained by removing a hydrogen atom from a carbon atom of a hydrocarbon compound having from 3 to 20 carbon atoms (unless otherwise specified), which may be aliphatic or alicyclic, and which may be saturated or unsaturated (e.g., partially unsaturated, fully unsaturated). Thus, the term "alkyl" includes the sub-classes alkenyl, alkynyl, cycloalkyl, cycloalkyenyl, cylcoalkynyl, etc., discussed below.

In the context of alkyl groups, the prefixes (e.g., C₃ to C₄, C₃ to C₆, etc.) denote the number of carbon atoms, or range of number of carbon atoms. For example, the term "C₃ to C₄ alkyl," as used herein, pertains to an alkyl group having from 1 to 4 carbon atoms. Examples of groups of alkyl groups include C₃ to C₄ alkyl ("lower alkyl") and C₂ to C₆ alkyl. Note that the first prefix may vary according to other limitations; for example, for unsaturated alkyl groups, the first prefix must be at least 2: for cyclic and branched alkyl groups, the first prefix must be at least 3; etc.

Examples of (unsubstituted) saturated alkyl groups include, but are not limited to, methyl (C₁), ethyl (C₂), propyl (C₃), butyl (C₄), pentyl (C₅) and hexyl (C₆).

Examples of (unsubstituted) saturated linear alkyl groups include, but are not limited to, methyl (C₁), ethyl (C₂), n-propyl (C₃), n-butyl (C₄), n-pentyl (amyl) (C₅) and n-hexyl (C₆).

Examples of (unsubstituted) saturated branched alkyl groups include iso-propyl (C₃), iso-butyl (C₄), sec-butyl (C₄), tert-butyl (C₄), iso-pentyl (C₅), and neo-pentyl (C₅).

Alkenyl: The term "alkenyl," as used herein, pertains to an alkyl group having one or more carbon-carbon double bonds. Examples of groups of alkenyl groups include C₃₋₄alkenyl, C₃₋₇alkenyl, C₂₋₁₂alkenyl.

Examples of (unsubstituted) unsaturated alkenyl groups include, but are not limited to, ethenyl (vinyl, -CH=CH₂) 1-propenyl(-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

Hydroxy-C₃ - C₈ alkyl: The term "hydroxy-C₃ - C₆ alkyl," as used herein, pertains to a C₃ - C₆ alkyl group in which at least one hydrogen atom (e.g., 1, 2, 3) has been replaced with a hydroxy group. Examples of such groups include, but are not limited to -CH₂OH, -CH₂CH₂OH, and -CH(OH)CH₂OH.

Hydrogen: -H. Note that if the substituent at a particular position is hydrogen, it may be convenient to refer to the compound or group as being "unsubstituted" at that position.

Aryl: The term "aryl," as used herein, pertains to a monovalent moiety obtained by removing a hydrogen atom from an aromatic ring atom of an aromatic compound, which moiety has from 3 to 20 ring atoms (unless otherwise specified). Preferably, each ring has from 5 to 7 ring atoms.

In this context, the prefixes (e.g., C₃₋₂₀, C₅₋₇, C₅₋₆, etc.) denote the number of ring atoms, or range of number of ring atoms, whether carbon atoms or heteroatoms. For example, the term "C₅₋₆aryl," as used herein, pertains to an aryl group having 5 or 6 ring atoms. Examples of groups of aryl groups include C₃₋₂₀aryl, C₅₋₂₀aryl, C₅₋₁₅aryl, C₅₋₁₂aryl, C₅₋₁₀aryl C₅₋₇aryl, C₅₋₆aryl, C₅aryl, and C₆aryl.

The ring atoms may be all carbon atoms, as in "carboaryl groups." Examples of carboaryl groups include C₃₋₂₀carboaryl, C₅₋₂₀carboaryl, C₅₋₁₅carboaryl, C₅₋₁₂carboaryl, C₅₋₁₀carboaryl, C₅₋₆carboaryl, C₅₋₆carboaryl, C₅carboaryl, and C₆carboaryl.

Examples of carboaryl groups include, but are not limited to, those derived from benzene (i.e., phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic ring, include, but are not limited to, groups derived from indane (e.g., 2,3-dihydro-1 H-indene) (C₉), indene (C₉), isoindene (C₉), tetraline (1,2,3,4-tetrahydronaphthalene (C₁₀), acenaphthene (C₁₂), fluorene (C₁₃), phenalene (C₁₃), acephenanthrene (C₁₅), and aceanthrene (C₁₆).

Alternatively, the ring atoms may include one or more heteroatoms, as in "heteroaryl groups." Examples of heteroaryl groups include C₃₋₂₀heteroaryl, C₅₋₂₀heteroaryl, C₅₋₁₅heteroaryl, C₅₋₁₂heteroaryl, C₅₋₁₀heteroaryl, C₅₋₇heteroaryl, C₅₋₆heteroaryl, C₅heteroaryl, and C₆heteroaryl.

Halo (or halogen): -F, -Cl, -Br, and -I.

Hydroxy: -OH.

### Other Terms

As used herein, the term "fouling" refers to the attachment and growth of microorganisms and small organisms to a substrate exposed to, or immersed in, a liquid medium, for example an aqueous medium, as well as to an increase in number of the microorganisms and/or small organisms in a container of the liquid medium.

Accordingly "foulers" or "microfoulers" are used interchangeably and refer to the organisms that foul a substrate. Fouling may occur in structures exposed to or immersed in fresh water as well as in sea water. In particular, the term may be used to refer to a solid medium or substrate exposed to, or immersed in sea water.

Accordingly, the term "antifouling" refers to the effect of preventing, reducing and/or eliminating fouling. Antifouling agents or compounds are also called "antifoulants".

An antifoulant compound is usually applied at a standard concentration which is the concentration that is effective for its purpose. Accordingly, a concentration less than or below the standard concentration is one where the antifoulant is not effective when it is used alone.

The term "substrate" as used herein refers to a solid medium such as surfaces of structures or vessels exposed to, or immersed in a liquid medium. The liquid medium may be fresh water or seawater and may be a body of water in a manmade container such as a bottle, pool or tank, or the liquid may be uncontained by any manmade container such as seawater in the open sea.

A "structure" as used herein refers to natural geological or manmade structures such as piers or oil rigs and the term "vessel" refers to manmade vehicles used in water such as boats and ships.

The "microorganisms" referred to herein include viruses, bacteria, fungi, algae and protozoans. "Small organisms" referred to herein can include organisms that commonly foul substrates exposed to, or immersed in, fresh water or seawater such as crustaceans, bryozoans and molluscs, particularly those that adhere to a substrate. Examples of such small organisms include barnacles and mussels and their larvae. Small organisms can also be called small animals. The term "organism" referred to herein is to be understood accordingly and includes microorganisms and small organisms.

The term "marine organism" as used herein refers to organisms whose natural habitat is sea water. The terms "marine microorganism" and "marine small organism" are to be understood accordingly.

Further, the term "microfouling" refers to fouling by microorganisms and the term "macrofouling" refers to fouling by organisms larger than microorganisms such as small organisms defined above.

The terms "biocide" or "biocidal compound" refer to compounds that inhibit the growth of microorganisms and small organisms by killing them. The terms "biostatic" or "biostatic compound" refer to compounds that inhibit the growth of microorganisms or small organisms by preventing them from reproducing and not necessarily by killing them.

The term "degradation" as used herein refers to the chemical breakdown or modification of a compound in water, preferably sea water.

The term "growth" as used herein refers to both the increase in number of microorganisms and small organisms, as well to the development of a small organism from juvenile to adult stages. Accordingly, biocides and biostatics can be applied as a treatment to a body of liquid or to a substrate surface to inhibit the growth of microorganisms and small organisms. As such, biocides and biostatics can be antifoulants and can prevent, reduce or eliminate biofilm formation.

Accordingly, the terms "bacteriocidal" and "bacteriostatic" refer to effects of compounds on bacteria.

The term "bioactivity" as used herein refers to the effect of a given agent or compound, such as a biocidal or biostatic compound, on a living organism, particularly on microorganisms or small organisms.

A "biofilm" is a complex aggregation of microorganisms, usually bacteria or fungi, marked by the excretion of a protective and adhesive matrix. Biofilms are also often characterized by surface attachment, structural heterogeneity, genetic diversity, complex community interactions, and an extracellular matrix of polymeric substances. Biofilms may also be more resistant to antibiotics compared to unaggregated bacteria due to the presence of the matrix.

The term "pharmaceutical" as it relates to a use, agent, compound or composition, refers to the medical treatment of a disease or disorder in humans or animals. Accordingly, a pharmaceutical compound is a compound used for the medical treatment of a disease or disorder in humans or animals.

As used herein, the term "standard concentration" as it pertains to an anti-fouling agent or compound, refers to the concentration at which the agent or compound is effective against microorganisms or small organisms at which it are directed when that agent or compound is used alone. Accordingly, the term "effective" means having a desired effect and the term "below standard concentration" refers to the level at which the agent or compound is not effective when used alone.

The inventors have carried out structure-function studies of the pharmaceutical compounds disclosed in US 11/265,833. The inventors have devised methodology to de-engineer these molecules and, based on their understanding of the structure activity relationship obtained from their studies, have synthesised a number of compounds whose antifouling potency with respect to the parent compound is substantially or entirely conserved, or even increased, whilst simplifying the structure of the compounds so to encourage rapid bacterial degradation in water.

Specifically, in order to gain a greater understanding of the structure-activity relationship of these pharmaceuticals, one of them, loperamide hydrochloride, was selected by the present inventors for more detailed studies.

Loperamide hydrochloride is slightly soluble in water, and soluble in methanol, isopropyl alcohol and chloroform. It is a white-yellow powder with a Mw of 513.51. Its pharmacodynamics in vertebrates is as follows: loperamide binds to opiate receptors in the gut wall, inhibiting the release of acetylcholine and prostaglandins. It is indicated for the symptomatic control of acute and chronic diarrhoea (Kleemann, 2001; Budavari, 1996).

The syntheses of structural derivatives were carried out to enable elucidation of the pharmacophore responsible for loperamide's observed antifouling effect. Further synthetic studies then lead to the simplification of the core structure and adjustment of physical and chemical properties.

Over thirty synthetic compounds derived from loperamide were synthesised by the present inventors and tested using three different bioassays to test for antibacterial and/or antifouling activity.

Antibacterial and/or antifouling activity was observed for several of the synthetic compounds, despite the compounds being significantly smaller and less complex than loperamide.

The present invention therefore provides a number of compounds derived from the de-engineering of loperamide, which compounds retain some or all of the bioactivity of loperamide. Indeed, some compounds surprisingly demonstrate higher levels of activity that loperamide.

In addition, the present inventors have found that small structural changes may alter the biodegradability of the compounds, suitably to increase the probability that they will biodegrade faster in the environment.

Thus, unlike existing molecules, including pharmaceuticals, suggested for use as an antifouling or antibacterial agent, the molecules identified by the present inventors are not only bioactive but structurally simple and biodegradable. Accordingly, these molecules will be useful for a variety of antifouling applications for the prevention of marine growth. For example, the molecules can be used as additives in marine antifouling coatings, biocides in the treatment of seawater and the prevention of fouling in processes using seawater, such as cooling towers and in desalination.

Furthermore, several bioactive compounds also demonstrated enhanced water solubility as compared to loperamide.

In preferred embodiments these molecules are incorporated into coatings in such a way that they are protected from premature degradation but released at a predetermined target time, after which they are degraded by bacteria in the environment. The skilled reader will be aware that the state of the art in polymer/coating chemistry provides several ways to deliver molecules in this way, depending on the requirements of the application.

In preferred embodiments, these compounds are incorporated into conventional antifouling coatings as antifouling agents for the prevention of marine growth. For example, the compounds can be blended into existing acrylate paints and are therefore practical alternatives to the current coating options. In particular, these compounds may be offered as environmentally safer alternatives to reduce use of existing booster biocides in existing coating formulations, as a replacement for poorly-degradable existing booster biocides, and/or augment existing coating formulations to improve performance. In this connection, a number of the compounds are oils and suitably compatible for incorporation into coatings, for example silicon-based foul-release coatings. In embodiments, this compatibility may impart the coatings with increased effectiveness such that the coated substrate benefits from additional protection.

Furthermore, these compounds may be applied in such way to reduce or replace copper/metal present in conventional antifouling coatings, thereby reducing the environmental impact of antifouling coatings.

Suitably, the compounds may be used in the removal of marine organisms in seawater treatment processes such as in ballast water treatment and for control of marine growth in cooling water and desalination processes. The compounds are particularly suited to processes where rapid degradation/removal of the active agent is necessary to prevent environmental contamination and for compliance purposes.

All of the compounds exhibiting antibacterial and/or antifouling activity are amides.

### Synthesis of Compounds

Several methods for the chemical synthesis of compounds of the present invention are described herein. These and/or other well known methods may be modified and/or adapted in known ways in order to facilitate the synthesis of additional compounds within the scope of the present invention.

The amides may be prepared in excellent yield from the corresponding carboxylic acid through the use of an amide coupling reagent as depicted below:

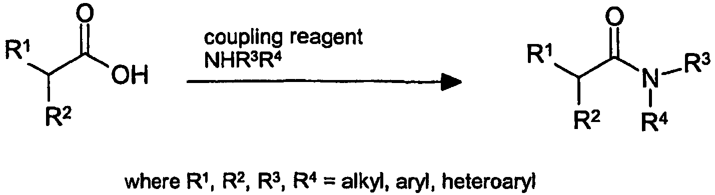

Alternatively, the amides may be prepared from the corresponding acid chloride in the presence of base:

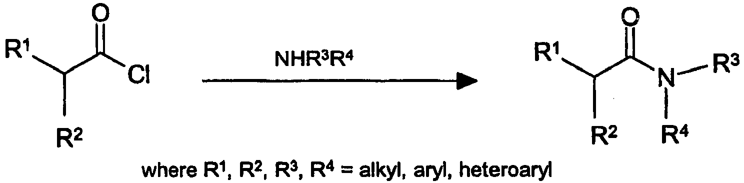

In addition, selected amides were synthesised through the ring opening of a dihydrofuraninium salt:

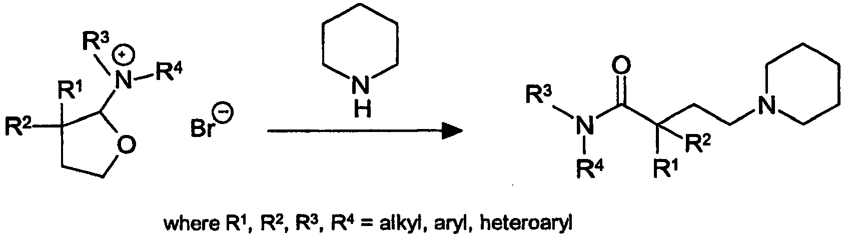

The structures of the compounds tested are given below.

| | |
|---|---|
| Compound 3.1 | 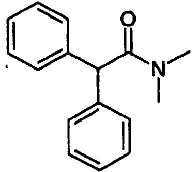 |
| Compound 3.2 | 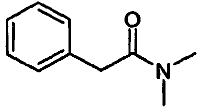 |
| Compound 3.3 | 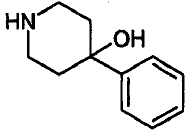 |
| Compound 3.4 | 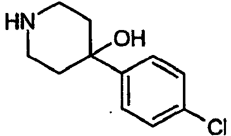 |
| Compound 4.1 | 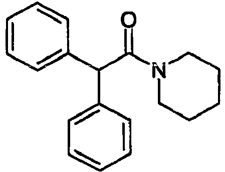 |
| Compound 4.2 | 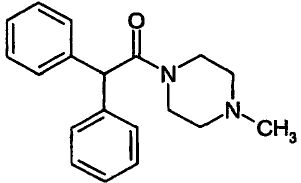 |
| Compound 4.3 | 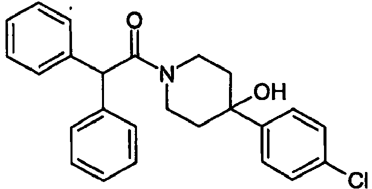 |
| Compound 4.4 | 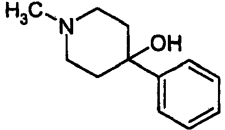 |
| Compound 4.5 | 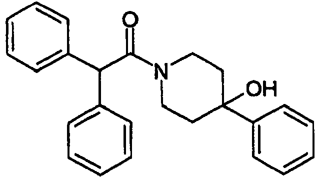 |
| Compound 4.6 | 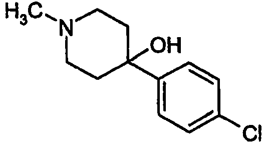 |
| Compound 4.7 | 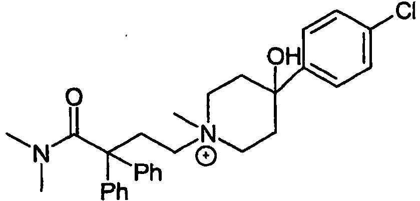 |
| Compound 5.1 | 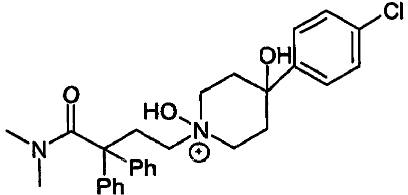 |
| Compound 5.2 | 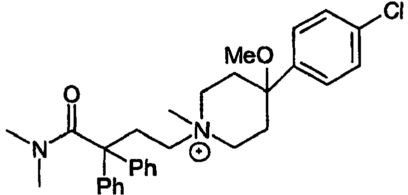 |
| Comnpound 5.2a | 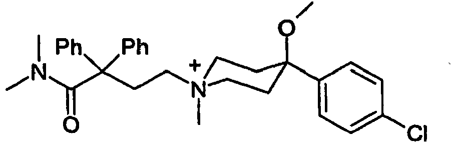 |
| Compound 5.2b | 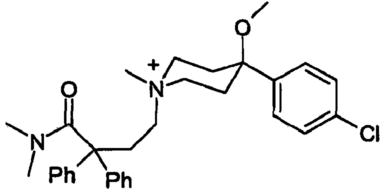 |
| Compound 5.3 | 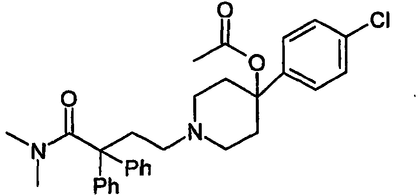 |
| Compound 8.1 | 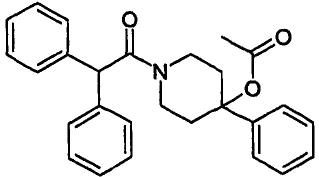 |
| Compound 9.1 | 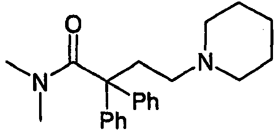 |
| Compound 9.2 | 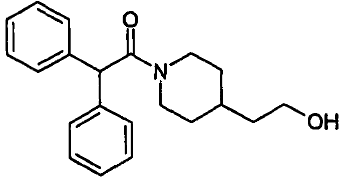 |
| Compound 9.3 | 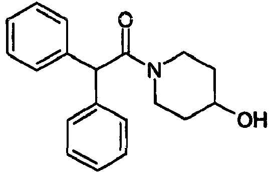 |
| Compound 10.1 | 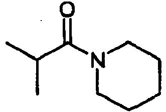 |
| Compound 10.2 | 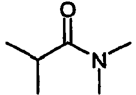 |
| Compound 10.3 | 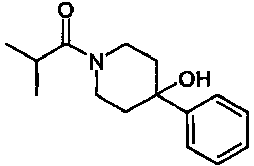 |
| Compound 10.4 | 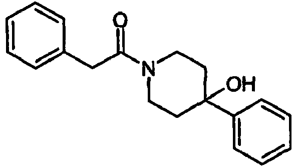 |
| Compound 10.5 | 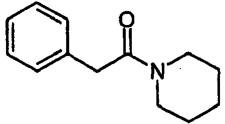 |
| Compound 10.6 | 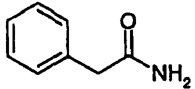 |
| Compound 10.7 | 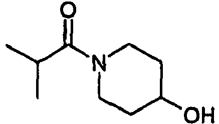 |
| Compound 10.8 | 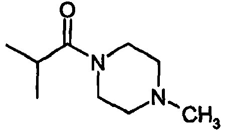 |
| Compound 10.9 | 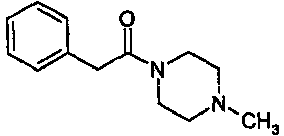 |
| Compound 11.1 | 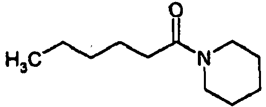 |
| Compound 11.2 | 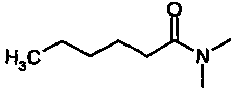 |
| Compound 11.3 | 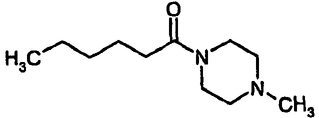 |
| Compound 11.4 | 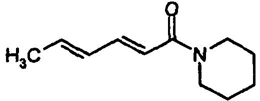 |
| Compound 12.1 | 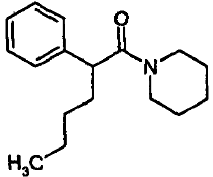 |
| Compound 12.2 | 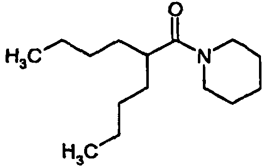 |
| Compound 12.3 | 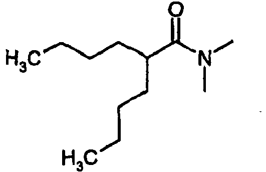 |
| Compound 12.4 | 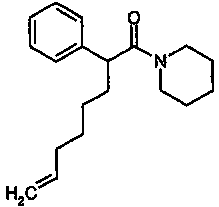 |
| Compound 12.5 | 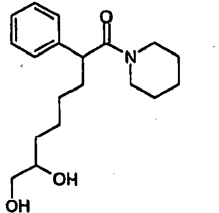 |
| Compound 12.6 | 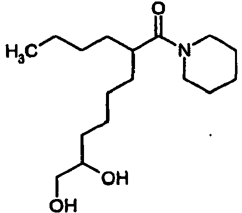 |
| Compound 12.7 | 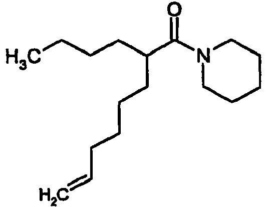 |
| Compound 12.8 | 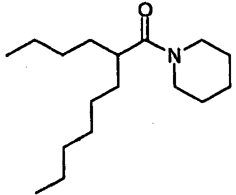 |
| Compound 12.9 | 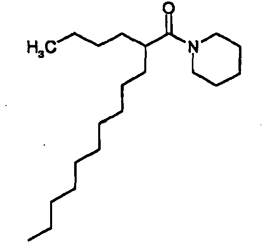 |
| Compound 12.10 | 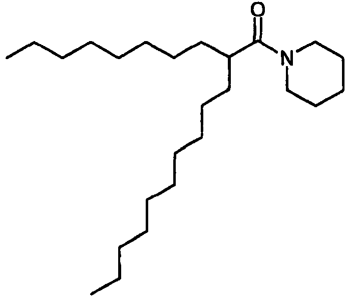 |
| Compound 12.11 | 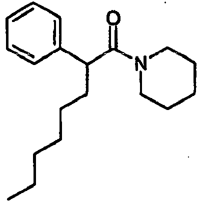 |
| Compound 12.12 | 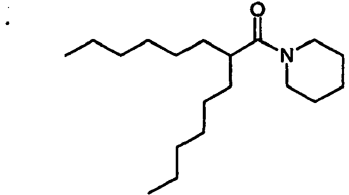 |

These compounds were tested for bioactivity against bacteria and/or barnacles.

### Synthetic Methods and Data for Selected Amide Derivatives

### Compound 4.1 - 2,2-Diphenyl-1-(piperidin-1-yl)ethanone

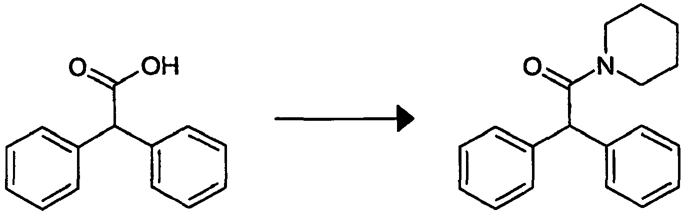

To a solution of carbonyldiimidazole (CDI) (0.35g, 2.14mmol) in dry, freshly distilled THF (10mL) under an argon atmosphere, was added diphenylacetic acid (454mg, 2.14mmol). The reaction mixture was allowed to stir at room temperature for 1 hour after which time it was cooled to 0°C and piperidine (0.2mL, 1.98mmol) in THF (5mL) was added. The reaction mixture was left at room temperature for 16 hours with stirring. The reaction mixture was poured onto aqueous saturated sodium hydrogen carbonate (20mL) and dichloromethane (25mL) was added. The organic layer was separated and the aqueous phase washed with dichloromethane (2 x 25mL). The combined organic extracts were dried with magnesium sulfate, filtered and the solvent was removed under reduced pressure.

The crude product was recrystallised from acetone and isolated as white crystals in 72% yield.
- ¹H NMR (CDCl₃):: δ 6 1.20 (m, 2H, C*H*₂); 1.48 (m, 4H, C*H*₂); 3.33 (m, 2H, *CH*₂); 3.55 (m. 2H, C*H*₂*)*; 5.15 (s, 1H, *C(O)CH)*; 7.16-7.24 (aromatic *CH).*
- ¹³C NMR (CDCl₃):: δ 24.5 (piperidine CH₂); 25.6 (piperidine *CH₂)*; *26.1* (piperidine CH₂); 43.4 (piperidine CH₂); 47.1 (piperidine *CH₂); 54.8* (C(O)CH); 126.9 (aromatic CH x 2); 128.4 (quaternary aromatic C); 128.5 (aromatic CH x 4); 129.0 (aromatic CH x 4); 139.7 (quaternary aromatic C); 170.0 (quaternary CO).
- EIMS:: *m*/*z* 279 (4%, M⁺); 226 (3%); 167 (27%); 112 (43%); 68 (100%).
- HREIMS:: *m*/*z* M⁺ 279.1628 (calculated for C₁₉H₂₁NO 279.1623)
- Melting Point:: 119.6-120°C
- Infrared νₘₐₓ (KBr):: 1637 s, 1493 w, 1435 m, 1357 w, 1278 w, 1250 m, 1219 m, 1135 w, 1017 m, 757 m, 709 s, 622 m cm⁻¹
- Anal.:: Calculated for C₁₉H₂₁NO: C, 81.68, H, 7.58, N, 5.01. Found C, 81.73, H, 7.31, N, 5.12.

### Compound 12.1 - 2-Phenyl-1-(piperidin-1-yl)hexan-1-one

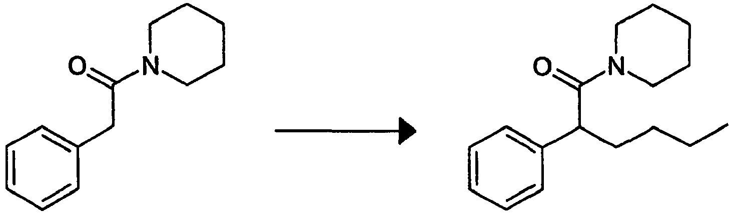

To a solution of 2-phenyl-1-(piperidin-1-yl)ethanone (1g, 4.92mmol) in dry, freshly distilled THF (10mL) under an argon atmosphere at 0°C, was added 2.5M n-butyllithium (3.94mL, 9.84mmol) followed by the dropwise addition of a solution of bromobutane (0.674g, .53mL, 4.92mmol) in THF (5mL). The reaction mixture was allowed to warm to room temperature overnight prior to the addition of 3N hydrochloric acid (10mL) and diethyl ether (10mL) and the layers were partitioned using a separatory funnel. The aqueous layer was isolated and washed with diethyl ether (2 x 20mL). The organic extracts were combined, dried with magnesium sulfate, filtered and the solvent removed *in vacuo* to give the crude product.

The title product was purified via flash silica column chromatography using a solvent gradient from 2% to 10% ethyl acetate in petroleum spirits and isolated as a colourless oil in 54% yield.
- ¹H NMR (CDCl₃):: δ 0.86 (t, J=8Hz, 3H, terminal *CH₃*); 0.99 (m, *CH₂*); 1.16 (m, *CH₂)*; 1.18-1.46 (m, *CH₂*); 1.51 (m, *CH₂*); 1.60 (m, *CH₂*); 1.69 (m, *CH₂*);2.09 (m, *CH₂*); 3.37 (t, J= 8Hz, *N-CH₂*); 3.42 (m, quaternary *CH); 3.68* (m, *CH₂*); 7.19-7.33 (m, aromatic *CH).*
- ¹³C NMR (CDCl₃):: δ 14.0 (CH₃); 22.7 *(CH₂);* 24.6 *(CH₂);* 25.5 *(CH₂);* 26.0 *(CH₂); 30.1 (CH₂);* 34.8 *(CH₂);* 43.1 *(N-CH₂);* 46.6 *(N-CH₂);* 48.8 (CH); 126.6 (aromatic CH); 127.8 (aromatic CH); 128.6 (aromatic CH); 140.9 (quaternary aromatic C); 171.3 (carbonyl C).
- ES+MS:: *m*/*z* 260 (M + H, 67%); 282 (M + Na, 100%).
- EIMS:: *m*/*z* 259 (6%, M⁺); 216 (16%, M⁺-Pr); 203 (47%, M⁺-Bu); 112 (100%); 91 (30%); 69 (32%).

### Compound 12.2 - 2-Butyl-1-(piperidin-1-yl)hexan-1-one

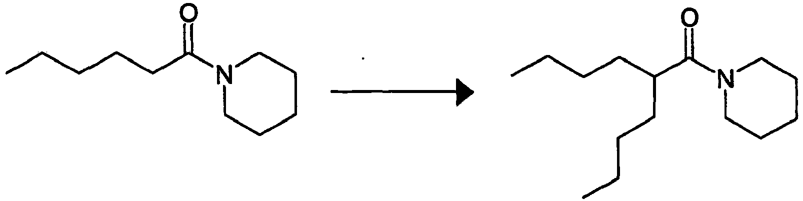

To a solution of di-iso-propylamine (0.92mL. 6.55mmol) in dry, freshly distilled THF (20mL) under an atmosphere of argon, was added 2.5M n-butyllithium (2.62mL, 6.55mmol) at -78°C. The reaction mixture was stirred for ten minutes at this temperature. To this solution was added the 1-(piperidin-1-yl)hexan-1-one (1.0g, 5.46mmol) and the solution stirred for one hour at -78°C. Bromobutane (0.59mL, 5.46mmol) was added and the solution stirred for one hour at -78°C before being allowed to reach room temperature overnight. 3N hydrochloric acid (10mL) was added to the reaction mixture, followed by the addition of diethyl ether (20mL) and the layers were partitioned using a separatory funnel. The aqueous layer was isolated and washed with diethyl ether (2 x 20mL). The organic extracts were combined, dried with magnesium sulfate, filtered and the solvent removed under reduced pressure to give the crude product.

The title product was purified via flash silica column chromatography (20% ethyl acetate in petroleum spirits, Rf 0.7) to give a colourless oil in 48% yield, based on recovered starting material.
- ¹H NMR (CDCl₃):: δ 0 0.87 (t, 6H, *J* = 7Hz, 2 x *CH₃*); 1.28 (m, *CH₂);* 1.42 (m, *CH₂*); 1.56 (m, *CH₂);* 1.65 (m, *CH₂*); 2.63 (m, 1H, *CH);* 3.48 (t, 2H, *J* = 6Hz, *NCH₂*); 3.6 (t, 2H, *J* =6Hz, *NCH₂).*
- ¹³C NMR (CDCl₃):: δ 14.1 (2 x CH₃); 22.9 (CH₂); 24.8 (CH₂); 26.0 (CH₂); 26.9 (CH₂); 29.9 (CH₂); 32.9 (CH₂); 40.7 (CH); 42.9 (N-CH₂); 46.8 (N-CH₂); 174.6 (quaternary CO).
- EIMS:: *m*/*z* 239 (6%, M⁺); 224 (2%, M⁺-Me); 210 (7%); 196 (24%); 183 (63%); 154 (13%); 140 (100%); 127 (32%); 112 (24%).
- HREIMS:: *m*/*z* M⁺ 239.2219 (calculated for C₁₅H₂₉NO 239.2249).

### Compound 4.2 - 1-(4-Methylpiperazin-1-yl)-2,2-diphenylethanone

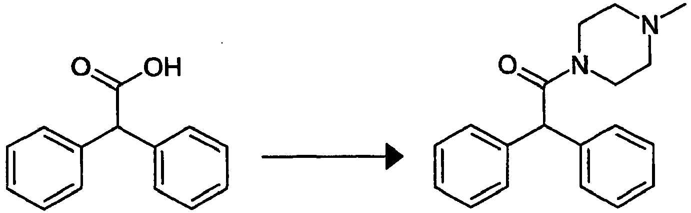

To a solution of CDI (0.77g, 4.72mmol) in dry THF (15mL) was added diphenylacetic acid (1g, 4.71 mmol). The reaction mixture was allowed to stir at room temperature for 1 hour after which time it was cooled to 0°C and N-methylpiperazine (0.5mL) in THF (10mL) was added. The reaction mixture was left at room temperature for 16 hours with stirring. The reaction mixture was poured onto aqueous sodium hydrogen carbonate (50mL) and dichloromethane (25mL) was added. The organic layer was separated and the aqueous phase washed with dichloromethane (2 x 25mL). The combined organic extracts were dried with magnesium sulfate, filtered and the solvent was removed under reduced pressure.

The product was recrystallised from acetone and isolated as white crystals in 88% yield.
- ¹H NMR (CDCl₃):: δ 1.79 (m, 2H, *CH₂);* 2.06 (m, 2H, *CH₂);* 2.18 (s, 3H, *NCH₃);* 2.32 (m, 2H, *CH₂);* 3.41 (m, 2H, *CH₂);* 3.67 (m, 4H, *CH₂);* 5.12 (s, 1H, *C(O)CH);* 7.14-7.34 (m, 10H, aromatic *H).*
- ¹³C NMR (CDCl₃):: δ 42.1; 45.8; 54.5; 54.7; 54.9; 121.4; 126.5; 127.1; 128.4; 128.5; 128.6; 129.0; 134.9; 139.2; 170.5 *(CO).*
- ESIMS:: *m*/*z* 295 (100%, [M+H]⁺); 296 (22%); 363 (16%).
- EIMS:: *m*/*z* 294 (100%, M⁺); 251(15%); 165 (74%); 127 (85%).
- HREIMS:: *m*/*z* M⁺ 294.1731 (calculated for C₁₉H₂₂N₂0 294.1732).
- Melting Point:: 129.5-130.7°C
- Infrared νₘₐₓ (KBr):: 1628 s, 1493 w, 1461 m, 1433 m, 1292 m, 1230 m, 1172 w, 1042 w, 745 w cm⁻¹
- Anal.:: Calc. for C₁₉H₂₂N₂0: C, 77.52, H, 7.53, N, 9.52. Found C, 77.19, H, 7.23, N, 9.45.

### Compound 9.1 - N, N-Dimethyl-2,2-diphenyl-4-(piperidin-1-yl)butanamide

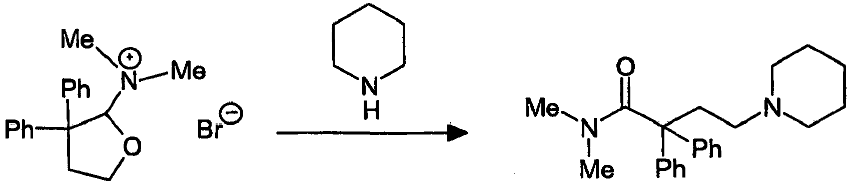

A mixture of piperidine (0.12mL, 1.25mmol), dihydro-N, *N*- dimethyl-3,3-diphenyl-2( 3H)-furaninium bromide (0.485g, 1.4mmol), sodium carbonate (0.25g, 2.35mmol) and N, N-dimethylformamide (12.5mL) was stirred at 80°C for eight hours. The reaction mixture was allowed to cool to room temperature and was stirred under an argon atmosphere for a further 16 hours. The solvent was removed under reduced pressure prior to the addition of water (10mL) and chloroform (10mL) and the layers were partitioned using a separatory funnel. The aqueous layer was isolated and washed with chloroform (2 x 20mL). The organic extracts were combined, dried with magnesium sulfate, filtered and the solvent removed *in vacuo* to give the crude product.

The title product was purified recrystallisation from methanol to give pale yellow crystals in 50% yield.
- ¹H NMR (CDCl₃):: δ 1.34 (m, *CH₂);* 1.48 (m, *CH₂*)*;* 1.68 (s, *CH₂*)*;* 2.03 (m, *CH₂*)*;* 2.27 (m, *CH₂);* 2.33 (m, *CH₂)*; 2.45 (m, *CH₂)*; 2.97 (s, 6H, *N-CH₃)*; 7.24-7.28 (m, aromatic CH); 7.33-7.41 (aromatic *CH).*
- ¹³C NMR (CDCl₃):: δ 24.4 *(CH₂);* 26.0 *(CH₂);* 39.1 *(CH₂);* 42.0 *(CH₂);* 54.6 *(CH₂); 56.5 (CH₂);* 59.7 (quaternary C); 126.6 (aromatic CH); 128.1 (aromatic CH); 128.3 (aromatic CH); 141.0 (quaternary aromatic C); 173.5 (carbonyl C).
- ESIMS:: *m*/*z 351* (100%, M + H).
- HR ESIMS:: *m*/*z* M + H 351.24243 (calculated for C₂₃H₃₁N₂O 351.24381).
- Melting Point:: 166.7-167.8°C.
- Infrared νₘₐₓ (KBr):: 3434 w, 3050 m, 2923 s, 2841 m, 1637 s, 1487 m, 1447 m, 1378 s, 1269 m, 1153 s, 1115 s, 1032 m, 859 w, 765 s, 741 m, 702 s, 639 s, 584 w, 471 w cm⁻¹
- Anal.:: Calc. for C₂₃H₃₀N₂O: C, 78.82, H, 8.63, N, 7.99. Found C, 78.75, H, 8.86, N, 8.07.

### Compound 5.2 - N,O - dimethyl Loperamide

To a mixture of Loperamide hydrochloride (250 mg, mmol), tetrabutylammonium iodide (0.1 eq, 18 mg, 0.049 mmol), 20% aq, NaOH (10 ml) in DCM (15 ml) was added methyl iodide (5 eq, 2.45 mmol, 152 µl). The mixture was stirred under argon at rt for 2h, washed with 1 N HCl (20 ml), water (20 ml) and dried over MgSO₄. It was then purified by a short silica gel column (10% MeOH in DCM). ESI-MS analysis showed a mixture of mono- and di-methylated product. The mixture was then stirred in excess methyl iodide (10 eq) under the same phase-transfer condition for 6h. it was then washed with 1N HCl (20 ml), water (20 ml) and dried over MgSO₄. Purification was achieved by flash chromatography (Silica gel, 0-10% MeOH in DCM) to give the two cis-trans isomers as the iodide salt.

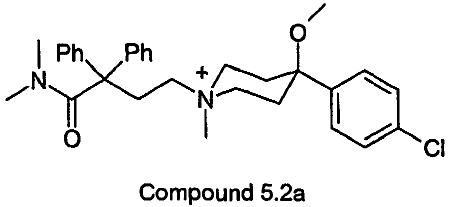

Compound 5.2a (135 mg, 44%): m.p. 152-154 °C: νₘₐₓ (cm⁻¹): 3455, 3055, 3030, 2943, 2830, 1626, 1492, 1450, 1391, 1261, 1141, 1066, 1011, 890, 827, 754, 730, 703: δ_{H} (MeOH-d₄, 400 MHz): 2.16-2.23 (2H, m, 2xCH-7β), 2.27-2.31 (2H, m, 2xCH-7a), 2.35 (3H, br s, amide NCH₃-α), 2.75-2.79 (2H, m, CH₂-3), 2.92 (3H, s, OCH₃), 3.01 (3H, brs, amide NCH₃-β), 3.01-3.05 (2H, m, CH₂-4), 3.05 (3H, s, N5-CH₃), 3.35-3.41 (4H, m, 2xCH₂-6), 7.39-7.53 (14H, m, Aromatic H); δ_{c} (MeOH-d₄, 100 MHz): 28.3 (CH₂-7), 36.0 (amide NCH₃-β), 36.9 (CH₂-3), 38.2 (amide NCH₃-α), 42.8 (N5-CH₃) 48.9 (OCH₃), 56.9 (CHᵣ6), 59.5 (C2), 67.0 (CH₂-4), 72.5 (C8), 127.4 (2xC11), 127.5 (2xC10), 127.9 (4xC14), 128.4 (2xC16), 128.7 (4xC15), 133.7 (C12), 138.6 (2xC13), 140.0 (C9), 173.1 (C=O): *m*/*z* (ESI): 505.6 (100%, [M]⁺), 437.5 (14%), 415.4 (8%), 301.4 (9%), 266.3 (33%), 242.5 (40%), 155.2 (38%): HRMS found [M]⁺ 505.2617, C₃₁H₃₈ClN₂O₂ requires [M]⁺ 505.2616: Anal. found C 56.35, H 5.76, N 4.01, caled for C₃₁H₃₈ClN₂O₂-1.5H₂O requires C 56.41, H 6.26, N 4.24.

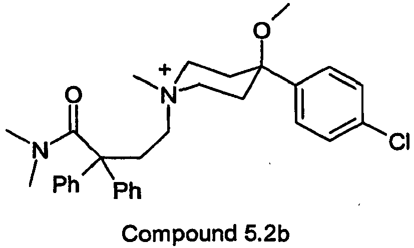

Compound 5.2b (42mg, 14%): m.p. 62-64°C; νₘₐₓ (neat, cm⁻¹) 3438, 3029, 2932, 1624, 1492, 1449, 1393, 1256, 1159, 1069, 1012, 917, 826, 703; δ_{H} (MeOH-d₄, 400 MHz): 1.80-1.88 (2H, m, 2xCH-7α), 2.07-2.11 (2H, m, 2xCH-β), 2.34 (3H, br s, amide NCH₃-α) 2.64-2.68 (2H, m CH₂-3) 2.94 (3H, s, OCH₃), 2.98 (3H, br s amide NCH₃-T), 3.08 (3H, s, N5-CH₃), 3.09-3.14 (2H, m, CH₂-4), 3.39-3.42 (2H, m 2xCH-6α), 3.48-3.54 (2H, m, 2xCH-6β), 7.31-7.52 (14H, m, Aromatic H); δ_{c} (MeOH-d₄, 100 MHz): 28.5 (CH₂-7), 36.0 (amide NCH3-β) 37.4 (CH₂-3), 38.2 (amide NCH₃-α), 49.0 (OCH₃), 51.5 (N5-CH₃), 55.0. (CH₂-4), 56.4 (CH₂-6), 59.7 (C2) 72.1 (C8), 127.3 (2xC11), 127.5 (2xC10), 127.9 (4xC14), 128.4 (2xC16), 128.8 (4xC15), 133.7 (C12), 138.8 (2xC13) 140.0 (C9) 172.9 C=O) *m*/*z* (ESI): 505.6 (100%, [M]⁺) 415.4 (3%) 266.3 (21%), 169.2 (8%) 155.2 (14%); HRMS found [M]⁺ 505.2610, C₃₁H₃₀ClN₂O₂ requires [M]⁺ 505, 2616.

CAS registry numbers for selected compounds are as follows: compound 9.1 - 95434-06-3; compound 4.7 - 251106-04-4; compound 5.1 - 217471-03-9; compound 5.3 - 296777-82-7; compound 4.4 - 4972-68-3; and compound 4.6 - 6653-07-2.

The remaining compounds were made by methods corresponding to those given above, with appropriate variation of starting materials.

### Biological Investigations - Methodology

Biological investigations focussed on the antibacterial activity of each compound as well as the effect of each compound on the viability of *Balanus amphifrite* nauplii (referred to as 'barnacle toxicity') and the settlement of *Balanus amphitrite* cyprids (referred to as 'anti-settlement behaviour'), the latter being particularly significant for the purposes of this study.

### Antibacterial Assay

Bacteria are very abundant in the marine environment. Many of them form biofilms on solid surfaces, which may be ship hulls or other submerged objects. Once formed, biofilms may modify the attachment behaviour of fouling macro-organisms such as barnacles, ship worms, etc (Maki et al., 1988; O'Connor, 1996; Maki et al., 2000; Huang and Hadfield, 2003). Microbial fouling involves the attachment of bacterial cells onto a surface, forming a biofilm. Following initial attachment of cells, multiple cell layers can be formed on top of this layer forming a biofilm. Organisms within biofilms are more resistant to antibiotics and cleaning agents.

For chemicals used in the environment, activity against bacteria has two implications. On one hand, bacterial activity is often responsible for breakdown of the antifouling agents in coatings, resulting in biodeterioration and poor performance. Microfouling bacteria are also a serious problem in fouling of membranes and heat exchanger surfaces. Novel antibacterial activity has important applications in water treatment systems. On the other hand, from an environmental perspective, persistent and strong antibacterial activity in chemical agents that are disposed into the marine environment can potentially result in impact on the natural micro-flora as well as development of more resistant strains of bacteria.

The effect of the compounds on marine bacteria found in microfouling biofilms was examined. The disc inhibition assay was used. This assay is a conventional method routinely used for screening antibacterial compounds to determine degree of susceptibility to antibacterial compounds. The diameter of the zone of inhibition is proportional to the degree of susceptibility of the bacterial strain. The compounds were tested against 13 strains of marine bacteria isolated from Singapore coastal waters.

13 strains of marine bacteria were isolated from fouling communities located in the coastal waters around Singapore. These were characterized in earlier studies and reported in Teo et al. (US Patent Application 11/265,833) and Choong et al (in prep). Table 1 provides the list of strains used in this assay. In addition, four reference bacterial strains were added tested: *Escherichia coli* (Strain K12, ATCC 15222), *E*. *coli* (strain DH5a), *Pseudomonas aeruginosa* (strain LMG 12228; ATCC 15692) and P. *putida* (strain KT2440; ATCC 47054). All the bacteria were also tested against five antibiotics: ampicillin (AMP), tetracycline (TETR), erythromycin (ERY), chloramphenicol (CHL) and streptomycin (STREP).

**TABLE 1 - selected biofilm strains isolated from various locations in Singapore marine waters, and their phylogenetic affiliation of 16S rRNA gene sequences.**

| Strain | Source of Isolation | Closely related species | Phylogenetic affiliation |
|---|---|---|---|
| S1 | PVC panel, Ponggol Marina | *Rhodovulum iodosum* | Alphaproteobacteria |
| S3 | Acrylic panel, Ponggol Marina | *Erythrobacter aquimaris* | Alphaproteobacteria |
| S4 | Panel, Ponggol Marina | *Bacillus algicola* | G(+) low G+C (Firmicutes) |
| S9 | Inside tube of *Vermitid,* Siglap Buoy | *Bacillus algicola* | G(+) low G+C (Firmicutes) |
| S10 | Inside sponge, Siglap Buoy | *Halobacillus trueperi* | G(+) low G+C (Firmicutes) |
| S14 | Under filamentous algae, Main Fairways Buoy | *Vibrio probioticus* | Gammaproteobacteria |
| S16 | Under barnacle, Main Fairways Buoy | *Pseudoalteromonas piscicida* | Gammaproteobacteria |
| S17 | Under spone, Main Fairways Buoy | *Gordonia terrae* | G(+) high G+C (Actinobacteria) |
| S18 | Under bryozoan, Main Fairways Buoy | *Microbacterium esteraromaticum* | G(+) high G+C (Actinobacteria) |
| S27 | On *Pomatoleios krausil,* St. John's Island | *Tenacibaculum lutimaris* | CFB group (Bacteroidetes) |
| S28 | Lim Chu Kang float | *Arthrobacter protophormial* | G(+) high G+C (Actinobacteria) |
| S29 | Under ascidian on rope, Changi fish farm | *Bacillus hwajinpoensis* | G(+) low G+C (Firmicutes) |
| S30 | Under ascidian on rope, Changi fish farm | *Bacillus borophilicus* | G(+) low G+C (Firmicutes) |

Disks comprising the compounds to be tested were prepared as follows. The antibacterial assay, the pure compounds were made up to a concentration of 2 mgml⁻¹ in DMSO. 25 µl of the stock was pipetted onto each 6 mm sterile disc (Macherey-Nagel #484000) to obtain 50 µg of compound per disc. Equivalent volume of DMSO was used inoculated for the control.

For all disk inhibition assays, bacterial cultures were grown in marine broth (Pronadisa #1217.00). Sterile swabs dipped into the cultures were used to inoculate Marine Agar plates with a bacterial lawn by smearing the culture over the surface. After inoculating the plates, discs with antibiotics, pharmaceuticals or control blanks were placed on each plate. Each concentration of antibiotic or pharmaceutical was tested with two replicates. After the disks were placed on the agar surface, plates were incubated overnight in the dark at 35°C. After incubation, plates were examined for zones of inhibition or clearing around control and treated disks. The diameter of any zone of clearing or inhibition was measured using Vernier calipers. There were two replicates per treatment, and the average was taken. There was no zone of inhibition around the control blanks in all the assays.

### Antifouling Assays

Pure compounds were suspended in DMSO and sonicated to obtain a stock solution of 50 mg ml⁻¹ in DMSO. This stock solution is stored at -20°C in 4ml amber screw cap vials. For the bioassays, a small volume of stock solution was added to 1 µm filtered seawater in a glass scintillation vial. This suspension was then sonicated around 10 minutes. Serial dilutions were generated to the required range of concentrations. Serial dilution of the equivalent amounts of DMSO in seawater was used as the control.

The barnacle bioassay method used follows from the method first introduced by Rittschof et al. (1992), and subsequently by other authors (Willemsen et al., 1998). This method is now standard practice for many antifouling screening of novel compounds.

Toxicity assays were modified from Rittschof et al. (1992). Stage II naupliar larvae used in tests were obtained from *Balanus amphitrite* adults collected from inter-tidal rock walls at Kranji mangrove, Singapore. Larvae were collected from a container of adults by attraction to a point source of light and transferred to 500 ml of fresh seawater. Next, larvae were re-concentrated with a fiber optic light and added to assays.

Duplicate assays were conducted in 2 ml glass vials (La Pha Pack® PN 11-14-0544) in 1ml of filtered seawater for 22 to 24 hours. The assays were repeated for confirmation. There were two sets of controls, a blank control consisting of 3 tubes of seawater only, and DMSO control consisting of the equivalent dilution series with DMSO without compound). For the compounds, there were 3 tubes of each for each test concentration. The assay was initiated by addition of barnacle naupliar in 50 µl of seawater. After 22 to 24 hours of incubation at 25-27°C solutions containing test animals were transferred to a Bogorov tray and scored as living or dead. Moribund larvae were scored as dead. Results were confirmed by repeating the assay. Data were combined and the concentration that caused 50 % mortality (LD50) was calculated by probit analysis using a basic computer program (Libermann, 1983). If data were not appropriate for probit analysis, the LD50 was estimated from graphed data.

Barnacle settlement assays were based on methodology from Rittschof *et al.* (1992). Barnacle larvae from field-collected adults were reared on an algal mixture of 1: 1 *v*/*v* of *Tetraselmis suecica* and *Chaetoceros muelleri* at 25°C, at approximately 5 x 10⁵ cells per ml density. On this regime, larvae metamorphose to cyprids in 5 days. These cyprids were aged at 4° C for 2-3 days, and 45-70% settlement after 24 hours (Willemsen et al., 1998).

Settlement tests were conducted in 7ml neutral glass vials (Samco® T1 *03N1)* 34mm by 23mm diameter, with 20-40 cyprids per well. Test solutions were made up to twice the required final concentration. 0.5 ml of test solution was added into each well, and cyprids were transferred into each well in 0.5 ml of seawater. Assays were done in triplicate. As before, there were two sets of controls, a blank control consisting 3 tubes of seawater only, and DMSO control consisting of the equivalent dilution series with DMSO without compound. After 24 hours, larvae that had attached and metamorphosed were enumerated and the result expressed as percentage settlement. Larvae not attached were scored as not settled. The assays were repeated and the concentration that caused 50 % settlement inhibition (ED50) was determined by pro bit analysis using a basic computer program (Lieberman, 1983), or by estimation from graphed data.

Lethal Dose (LD50, toxicity) and Effective Dose (ED50, antisettlement) values are presented below. Compounds for which both the LD50 and ED50 were greater than 50µgmL⁻¹, which was the highest concentration tested, have not been included in the table below, these are considered to be 'inactive'.

The Therapeutic Ratio, or LD50/ED50, is used to assess the effectiveness of the compound in relation to its toxicity.

### Biological Investigations - Results

The results are summarised in Tables 2(a) and (b). Where there is activity, a clear zone is observed around the disks. The width of the observed inhibition zone is a function of the potency of the compound and its solubility (that is, the extent of diffusion of the compound out of the disk, and into the agar medium). None of the compounds showed any activity against strain S14 and only one compound, compound 5.2, had activity against strain S1.For most strains, where there was activity detected for loperamide and Imodium®, activity was also detected for compounds 12.1, 12.2 and in most instances 4.1 and the loperamide analogues 4.7, 5.2, 5.3. The removal of branching at the alpha carbon (compounds 3.2, 10.5, 11.1, 11.2, 11.3 and 11.4) or the replacement of both phenyl rings with a methyl substituent (compounds 10.1 and 10.2) resulted in an observed loss of antibacterial activity, except for S16 bacteria strain. Strain S16 was susceptible to a very different array of compounds compared to the other compounds suggesting that it may be responding to a different pharmacophore. Unlike all the other strains, S16 was susceptible to the compounds with a single alkyl chain (compounds 11.1, 11.2 and 11.4). From sequencing results, S16 is affiliated to *Pseudoalteromonas* sp.

For all of the compounds, no inhibition was observed against the reference bacterial strains. This suggested that the antibacterial activity observed from these compounds may be lower than conventional antibiotics. Nevertheless, this is not regarded as particularly important because activity levels lower than conventional antibiotics can still provide useful results.

**Table 2a: Activity of compounds against local isolates of biofilm bacteria**

| Compounds | BACTERIA STRAINS | | | | | | | | | | | | |
|---|---|---|---|---|---|---|---|---|---|---|---|---|---|
| | 1 | 3 | 4 | 9 | 10 | 14 | 16 | 17 | 18 | 27 | 28 | 29 | 30 |
| 3.1 | - | + | - | - | - | - | - | - | - | - | - | - | - |
| 3.4 | - | - | + | + | - | - | + | - | - | - | - | - | - |
| 4.1 | - | ++ | + | + | + | - | - | + | + | + | + | - | - |
| 4.3 | - | + | - | - | - | - | - | - | - | - | - | - | - |
| 4.4 | - | - | - | - | - | - | - | - | - | - | - | - | - |
| 4.5 | - | ++ | - | - | - | - | - | - | - | - | - | - | - |
| 4.6 | - | - | - | + | - | - | + | - | - | - | - | - | - |
| 4.7 | - | ++ | ++ | ++ | ++ | - | + | + | + | - | ++ | ++ | + |
| 5.1 | - | + | - | - | - | - | + | + | - | - | - | - | - |
| 5.2 | + | ++ | ++ | ++ | ++ | - | + | + | +++ | - | ++ | ++ | ++ |
| 5.3 | - | ++ | + | + | ++ | - | - | + | + | - | + | + | + |
| 9.3 | - | - | - | - | + | - | + | - | - | - | - | - | - |
| 9.1 | - | - | - | + | - | - | - | - | + | - | - | - | - |
| 10.4 | - | + | - | + | - | - | + | - | - | + | - | - | - |
| 10.5 | - | - | - | - | - | - | ++ | - | - | - | - | - | - |
| 11.1 | - | - | - | - | - | - | ++ | - | - | - | - | - | - |
| 11.2 | - | - | - | - | - | - | + | - | - | - | - | - | - |
| 12.1 | - | ++ | + | + | + | - | - | + | + | + | + | + | + |
| 12.2 | - | ++ | + | + | + | - | - | + | ++ | + | + | + | + |
| LOP | - | +++ | ++ | ++ | ++ | - | - | ++ | +++ | ++ | ++ | ++ | + |
| IMD | - | ++ | ++ | ++ | + | - | + | + | + | + | ++ | ++ | + |

**Table 2b: Activity of further compounds against local isolates of biofilm bacteria**

| COMPOUNDS | BACTERIA STRAINS | | | | | | | | | | | | |
|---|---|---|---|---|---|---|---|---|---|---|---|---|---|
| | 1 | 3 | 4 | 9 | 10 | 14 | 16 | 17 | 18 | 27 | 28 | 29 | 30 |
| 12.3 | - | - | - | - | - | - | - | - | - | - | - | + | - |
| 12.4 | - | +++ | + | + | + | - | - | + | ++ | + | - | + | - |
| 12.5 | - | - | - | - | - | - | - | - | - | - | - | + | - |
| 12.6 | - | - | - | - | - | - | - | - | - | - | - | - | - |
| 12.7 | + | +++ | ++ | ++ | ++ | - | - | ++ | +++ | + | + | + | + |

The zone of inhibition represents a clear area around each disk where no bacteria growth was observed.
(-) No zone was observed. Test compound exhibited no activity against bacteria in the disk assay.
(+) 2-5mm zone of inhibition around each disk was observed.
(++) 5-10 mm zone was observed.
(+++) Greater 10mm zone observed.
LOP= loperamide pure compound;
IMD= Imodium® suspended in DMSO to give a loading of 50ug active ingredient.

Also tested were 5 conventional antibiotics. The results are presented in Table 2(c). In general, the compounds were less potent than the antibiotics tested, and the pattern of activity was different.

**Table 2 (c) Activity of conventional antibiotics.**

| | AMP | | TETR | | ERY | | CHL | STREP | |
|---|---|---|---|---|---|---|---|---|---|
| Bacterial strains | 2ug | 10ug | 5ug | 30ug | 5ug | 15ug | 30ug | 10ug | 25ug |
| 1 | ++ | ++ | - | - | - | - | ++ | ++ | +++ |
| 3 | - | - | - | - | ++ | ++ | +++ | - | - |
| 4 | +++ | +++ | + | ++ | +++ | +++ | +++ | + | ++ |
| 9 | + | +++ | + | ++ | +++ | +++ | +++ | + | + |
| 10 | +++ | +++ | + | + | ++ | +++ | +++ | + | + |
| 14 | + | + | - | + | - | + | ++ | + | + |
| 16 | + | + | - | - | + | + | ++ | + | ++ |
| 17 | - | - | - | - | +++ | +++ | +++ | + | ++ |
| 18 | + | ++ | - | + | + | ++ | ++ | + | ++ |
| 27 | ++ | +++ | - | + | ++ | ++ | +++ | - | - |
| 28 | + | ++ | - | + | +++ | +++ | +++ | + | ++ |
| 29 | +++ | +++ | + | ++ | +++ | +++ | +++ | + | ++ |
| 30 | +++ | +++ | + | + | ++ | ++ | ++ | + | ++ |
| REF 1 | - | + | + | ++ | - | + | ++ | + | ++ |
| REF 2 | - | + | + | ++ | - | + | ++ | - | - |
| REF 3 | - | - | + | ++ | + | + | ++ | + | ++ |
| REF 4 | - | + | ++ | ++ | + | + | ++ | + | ++ |

REF 1: *Escherichia coli* (Strain K12; ATCC 15222)
REF 2: *Escherichia coli* (Strain DH5a)
REF 3: *Pseudomonas aeruginosa* (strain LMG 12228; ATCC 15692)
REF 4: *Pseudomonas putida* (Strain KT2440; BCRC 10459)
AMP = Ampicillin
TETR = Tetracycline
ERY = Erythromycin
CHL = Chloramphenicol
STREP = Streptomycin

The LD50 and ED50 values for the compounds are presented in Table 3(a). For some of the compounds, the LD50 and ED50 were greater than 50 µg/ml (which is the highest concentration tested) and so, whilst activity is present, generally no further testing of those compounds was undertaken.

**Table 3(a): Bioactivity against barnacle larvae**

| Compound Ref | LD50^{#} | ED50* | TR | |
|---|---|---|---|---|
| 9.1 | 1.68 | 0.07 | 24 | |
| 4.1 | 50 | 2.35 | 21.27 | * |
| 12.3 | 50 | 2.76 | 18.12 | * |
| 4.5 | 50 | 6.48 | 7.72 | * |
| 12.4 | 3 | 0.47 | 6.38 | |
| 12.7 | 1.16 | 0.26 | 6.38 | |
| 12.1 | 9.11 | 1.5 | 6.07 | |
| 12.2 | 9.83 | 2 | 4.92 | |
| Loperamide | 1.56 | 0.37 | 4.22 | |
| 10.4 | 50 | 15 | 3.33 | * |
| 3.1 | 50 | 15.1 | 3.31 | * |
| 4.4 | 12.35 | 5.98 | 2.07 | |
| 5.3 | 0.66 | 0.45 | 1.47 | |
| 9.2 | 49.24 | 50 | 0.98 | * |
| 4.3 | 0.82 | 1 | 0.82 | |
| 4.6 | 2.41 | 7.92 | 0.30 | |
| 4.2 | 14.32 | 50 | 0.29 | * |
| 3.4 | 8.41 | 43.93 | 0.19 | |

| | | | | |
|---|---|---|---|---|
| * Where the LD50, ED50 was >50, a nominal value of 50 was assigned for estimation of the therapeutic ratio (TR), hence the actual therapeutic ratio is likely to be higher/lower than the estimated. In fact, for compound 4.1, further experiments have shown that the LD50 is 100, and the TR is 42.55. For compound 11.3, further experiments showed the LD50 to be 88.28. For all the remaining compounds, whilst activity is present, values of LD50 and ED50 greater than 50 are generally not included in the table. ^{#}fhe highest concentration tested was 25 µg/ml. | | | | |

The results of tests for a further 5 compounds are set out in table 3(b) and 3(c) below.

**Table 3(b): Toxicity of further compounds against barnacle larvae**

| **Compound** | **LD₅₀^{#}** |
|---|---|
| 12.8 | 3.35 |
| 12.9 | >25 |
| 12.10 | >25 |
| 12.11 | >25 |
| 12.12 | 0.2 - 1 |

For the anti-settlement tests, the values given below are, averaged over two trials, the percentage of cyprids that settled after 24 hours incubation.

**Table 3(c): Bioactivity of further compounds against barnacle larvae**

| TREATMENTS /Test Conc | Control (seawater only) | Control (DMSO) | 12.2 | 12.8 | 12.9 | 12.10 |
|---|---|---|---|---|---|---|
| No compound | 30.43 | 35.44 | | | | |
| 0.2 ug/ml | | | 8.94 | 25.82 | 28.14 | 12.5 |
| 1 ug/ml | | | 8.42 | 8.62 | 10.56 | 13.00 |
| 5 ug/ml | | | 8.37 | 0 | 1.94 | 11.40 |
| 25 ug/ml | | | 0 | 0 | 1.32 | 17.88 |

### Discussion of Results

Conceptually, compounds 3.1 and 4.6 can be considered as the two major fragments obtained when the loperamide parent structure is divided into two.

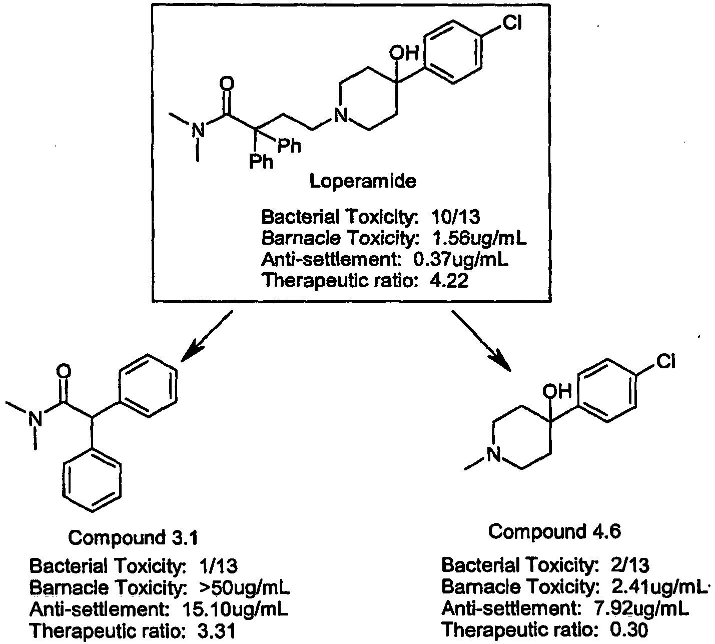

Biological potency is present in both fragments as bioassays demonstrated that both compounds 3.1 and 4.6 retained detectable biological activity.

The therapeutic ratio value for compound 4.6 was less than one, indicating toxicity, whereas the TR for compound 3.1 was greater than one. Compounds with high TR values are deemed to have good potential as antifouling compounds because they elicit an anti-settlement effect at sub-lethal concentrations.

Compound 3.1 is a known pesticide with the common name diphenamid (commercial names include Dymid™ and Enide™) and its microorganism-facilitated biodegradation in soil has been documented (Avidov 1990; Avidov 1988). Kugler et al. have previously filed a patent covering the use of a variety of insecticides and herbicides, including diphenamid, in antifouling compositions (Kugler, United States Patent Number 5,990,043).

Given its desirable TR value, preliminary structure-function studies were carried out from compound 3.1 and these revealed several Interesting trends.

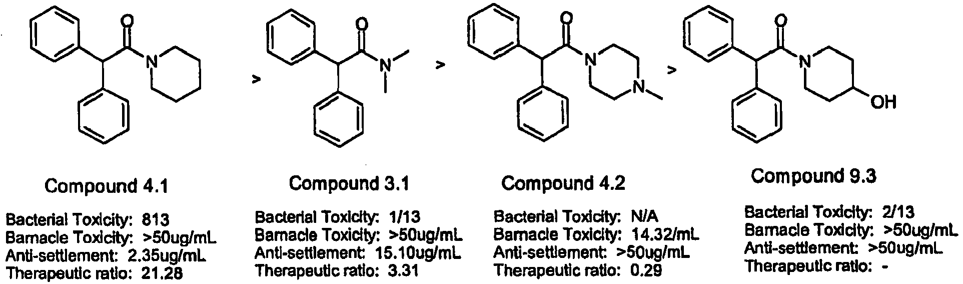

Bacterial toxicity is given as the number of strains inhibited over the total strains tested. Barnacle toxicity is given as the LD_{50,24h} (µg/mL) for toxicity to Stage II nauplii of Balanus amphitrite. Anti-settlement activity is the ED_{50, 24h} (µg/mL) against settlement of cyprids of Balanus amphitrite.

Incorporation of the amide nitrogen into a six membered ring (piperidine) structure increased antibacterial and anti-settlement activity (compound 3.1 versus compound 4.1) whereas incorporation into an N-methylpiperazine ring reduced bacterial toxicity, increased barnacle naupliar toxicity but eliminated anti-settlement behaviour (compound 4.2), while the addition of alcohol functionality on the piperidine ring further reduced activity (compound 9.3).

Compound 4.1 displayed anti-settlement activity but no observable naupliar toxicity, giving rise to a large TR value. Compound 4.1 was therefore utilised as a lead for further structural simplification. The activity and high TR for compound 4.1 is surprising given that it has been shown to display antispasmodic activity (Cheney, 1952), which is unrelated to the activity demonstrated herein.

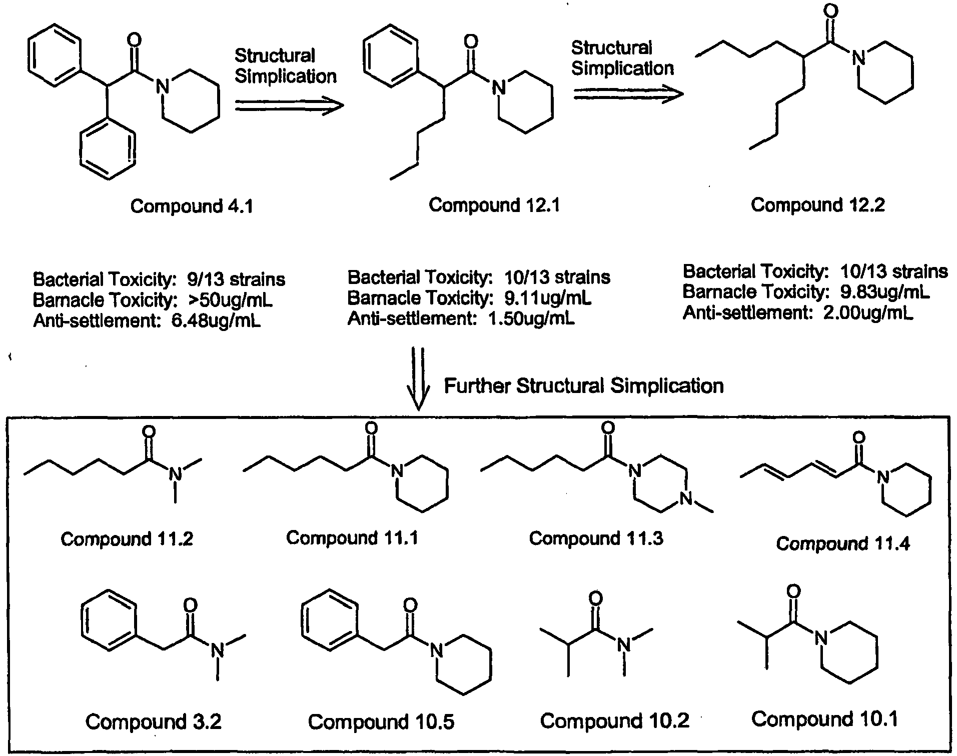

Bacterial toxicity is given as the number of strains inhibited over the total strains tested. Barnacle toxicity is given as the LD_{50,24h} (µg/mL) for toxicity to Stage II nauplii of Balanus amphitrite. Anti-settlement activity is the ED_{50,24h} (µg/mL) against settlement of cyprids of Balanus amphitrite.

Replacing both of the phenyl groups with a single n-butyl group (compound 11.1) or an unsaturated butyl group (compound 11.4) and a hydrogen atom resulted in compounds with reduced potency. In general, it appears that the removal of branching at the alpha carbon through removal of one phenyl group (as in compounds 3.2,10.5,11.1,11.2,11.3 and 11.4) or replacement of both with a methyl group (as in compounds 10.1 and 10.2) resulted in a net loss of activity.

However, the replacement of one or both of the phenyl rings in compound 4.1 with n-butyl chains (for example, compounds 12.1 and 12.2) did not result in an appreciable loss of activity.

The removal of the piperidine ring in compound 12.3 resulted in loss of antibacterial activity but bioactivity against barnacle larvae was retained. Addition of -OH groups on the alkyl chain results in reduced activity. Addition of the double-bond on the alkyl chain resulted in no major change in activity compared to 12.1 and 12.2.

The most potent compounds in the series, where the LD50 and ED50 were less than *10µg*/*mL* and TR values greater than one, are the compounds 9.1, 5.3, 12.1, 12.2, 12.4, 12.7 and 12.8.

The LD50 and ED50 for compounds 4.3 and 4.6 were also less than 10µg/mL but their TR values are less than one, indicating that these compounds are more toxic than repellent. Of the active compounds, compounds 12.1 (for previous synthesis see Marlensson, 1960), 12.2, 12.4, 12.7 and 12.8 represent the most promising antibacterial and/or antifouling agents.

Of the compounds with high therapeutic ratio against barnacles, the compounds 12.2, 12.4 and 12.7 have the simplest chemical form. Both these molecules were also active against bacteria, and their activities are comparable to (or better than) loperamide. These compounds are much smaller and simpler in structure than most existing antifouling compounds, and lack any halogenated and aromatic ring structures. These compounds are therefore environmentally benign antifouling agents.

The compounds of the present invention have the considerable advantage of providing the antifouling coating market with an organic alternative to the existing technology which relies heavily on the addition of copper to obtain significant antifouling effects. The compounds we have developed may be used as cheap, easy to prepare additives that do not contain metals and therefore have reduced toxicity in marine environment. In particular, compound 12.2 lacks any halogen or aromatic ring structures.

The compounds can be blended into existing acrylate paints and are therefore practical alternatives to the current coating options. Furthermore, due to their simple structure the compounds are attractive candidates for degradation via bacterial means in the marine environment and are less likely to accumulate and pose a health risk in the future. In addition, given that existing organic biocides such as Diuron® and Sea-Nine® have been shown to bioaccumulate and cause detrimental effects in the marine environment, the compounds of the present invention represent a valuable alternative to traditional metal-based additives.

### REFERENCES

A number of patents and publications are cited above in order to more fully describe and disclose the invention and the state of the art to which the invention pertains. Full citations for these references are provided below.
Avidov, E.; Aharonson, N.; Katan, J. 'Involvement of soil microorganisms in the accelerated degradation of diphenamid', Weed Science 1990, 38(2), 186-93.
Avidov, E.; Aharonson, N.; Katan, J. 'Accelerated degradation of diphenamid in soils and means for its control', Weed Science 1988, 36(4), 519-23.
Bellas J, A Hilvarsson, G Birgersson & A Granmo, 2006. 'Effects of medetomidine, a novel antifouling agent, on the burrowing Abra nitida (Moiler)', Chemosphere 65: 575-582.
Budavari, S.; O'Neil, M. J.; Smith, A. ; Heckelman, P. E.; Kinneary, J. F. The Merck Index: An Encyclopedia of Chemicals, Drugs and Biologicals, Twelfth Edition, Whitehouse Station, New Jersey, 1996.
Cheney, L. C.; Wheatley, W. B.; Speeter, M. E.; Byrd, W. M.; Fitzgibbon, W. E.; Minor, W. F.; Binkley, S. B. 'Basic amides as antispasmodic agents. I.', J. Org. Chem. 1952, 17, 770-7.
Choong, A. M-F, JS Maki, Juriani Tan bte Ikhwan, C-L Chen, D Rittschof & S L-M Teo (in prep). Pharmaceuticals as antifoulants: inhibition of growth and effects on adhesion of marine bacteria.
Harino H, Y Yamamoto, S Eguchi, S Kawai, Y Kurokawa, T Arei, M Ohji, H Okamura & N Miyazaki, 2007. Concentrations of Antifouiing Biocides in Sediment and Mussei Samples Collected from Otsuchi Bay, Japan. Arch. Environ. Contam. Toxico/. 52: 179-188.
Huang S-Y, Michael G. Hadfield. 2003. Composition and density of bacterial biofilms determine larval settlement of the polychaete Hydroides e/egans. Marine Ecology Progress Series 260: 161-172.
Kleemann, A.; Engel, J. (eds), Pharmaceutical Substances: Syntheses, Patents, Applications, Fourth Edition, Thieme, New York, 2001, pp. 1186-1187.
Kugler, M.; Londershausen, M.; Schrage, H.; Uhr, H.; Kunisch, F., 'Anti-fouling Compositions', US Patent File 5,990,043, 23 November 1999.
Libermann HR, 1983. Estimating LD50 using the probit technique: a basic computer program. Drug Chem Toxicol 6:111-116.
Maki JS, D Rittschof, JD Costlow & R Mitchell, 1988. Inhibition of attachment of larval barnacles, Balanus amphitrife, by bacterial surface films. Mar Biol. 97: 199-206.
Maki JS, L Ding, J Stokes, JH Kavouras & D Rittschof, 2000. Substratum/bacterial interactions and larval attachment: films and exopolysaccharides of Ha/omonas marina (ATCC 25374) and their effect on barnacle cypris larvae, Balanus amphitrite Darwin. Biofouling 16: 159-170.
Marlensson, 0.; Nilsson, E. 'Alkylation-type substitution and ester condensation at the a -position of N,N-disubstituted amides of arylacetic acids', Acta Chemica Scandinavica 1960, 14, 1129-50.
O'Connor, N.J., and Richardson, D.L. 1996. Effects of bacterial films on attachment of barnacle (Ba/anus Improvisus Darwin) larvae: laboratory and field studies. Journal of Experimental Marine Biology and Ecology 206:69-81.
Rittschof D, AS Clare, DJ Gerhart, Sr Avelin Mary & J Bonaventura, 1992. Barnacle in-vitro assays for biologically active substances: toxicity and settlement inhibition assays using mass cultured Ba/anus amphitrife amphitrite Darwin. Biofouling 6: 115-122.
Rittschof D, 2000. Natural product antifoulants: one perspective on the challenges related to coatings development. Biofouling 15: 199-127.
Rittschof D, 2001. Natural product antifoulants and coatings development. In: McClintock J, Baker P (eds) Marine Chemical Ecology, CRC Press, NY, pp 543-557. Rittschof Dan, Chien-Houng Lai, Lai-Mun Kok & Serena Lay-Ming Teo (2003). Pharmaceuticals as antifoulants: concepts and principles. Biofouling Vol. 19 (Supplement), pp. 207-212.
US Patent File 11/265,833. Method for biocidal and/or biostatic treatment and compositions therefore. S. L-M Teo, A M-F Choong, Sin T-M, D Rittschof, JS Maki. USPTO, 2006.
Voulvoulis, N, 2006. Antifouling paint booster biocides: occurrence and partitioning in water and sediments. In: Konstantinou, I. K. (ed). Antifouling Paint Biocides. The Handbook of Environmental Chemistry, Volume 5, Part 0, pp.155-170. Springer-Verlag Berlin-Heidelberg.
Willemsen PR, K Overbeke, A Suurmond, 1998. Repetitive testing of TBTO, Sea-Nine 211 and famesol using Balanus amphitrite (Darwin) cypris larvae: variability in larval sensitivity. Biofouling 12:133-147.

## Claims

1. Use of a compound of formula (I) in a method of reducing or preventing fouling 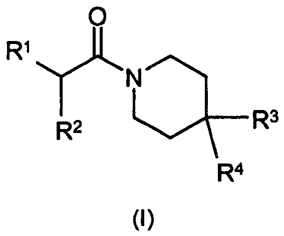 wherein
R¹ and R² are independently selected from optionally substituted aryl and optionally substituted C₃ to C₁₂ alkyl; and
R³ and R⁴ are independently selected from hydroxyl, optionally substituted C₁ to C₆ alkyl, optionally substituted phenyl and H.

2. Use according to claim 1, wherein at least one of R¹ and R² is C₃₋₅ alkyl.

3. Use according to claim 2, wherein at least one of R¹ and R² is n-butyl.

4. Use according to any one of the preceding claims, wherein at least one of R¹ and R² is C₅ to C₁₅ aryl, preferably phenyl.

5. Use according to claim 4, wherein (a) one of R¹ and R² is phenyl and the other is n-butyl, or (b) R¹ and R² are the same.

6. Use according to claim 5, wherein (a) R¹ and R² are both n-butyl, or (b) R¹ and R² are both phenyl.

7. Use according to any one of the preceding claims, wherein one or both of R¹ and R² are unsubstituted.

8. Use according to any one of the preceding claims, wherein (a) one of R³ and R⁴ is hydroxyl and the other is H, (b) both of R³ and R⁴ are H, or (c) one or both of R³ and R⁴ is substituted C₁ - C₆ alkyl.

9. Use according to claim 8, wherein one or both of R³ and R⁴ is hydroxy-C₁ - C₆ alkyl.

10. Use according to claim 9, wherein one of R³ and R⁴ is -CH₂CH₂OH and the other one of R³ and R⁴ is H.

11. Use according to claim 1, wherein the compound is selected from compounds 12.2, 12.1, 12.7, 12.4, 12.8, 12.9, 12.10, 12.11, 12.12, 4.1, 4.3, 9.2, 9.3 and 4.5.

12. Use according to any one of the preceding claims, wherein the method of preventing or reducing fouling is (a) a method of preventing or reducing microfouling or macrofouling; (b) a method of reducing or preventing biofilm formation; (c) a method of reducing or preventing biofilm formation by one or more of bacteria, fungi, algae and protozoans; (d)a method of reducing or preventing biofilm formation by bacteria; (e) a method of reducing or preventing macrofouling by one or more of crustaceans, bryozoans and molluscs; or (f) a method of reducing or preventing macrofouling by one or more of barnacles and mussels and their larvae.

13. A method of preventing or reducing fouling of a substrate, wherein the method comprises the step of applying to the substrate a compound as defined in any one of claims 1 to 11.

14. An antifouling composition or coating composition comprising a compound as defined in any one of claims 1 to 11.

## Patentansprüche

1. Verwendung einer Verbindung der Formel (I) in einem Verfahren zur Reduktion oder Prävention von Fouling 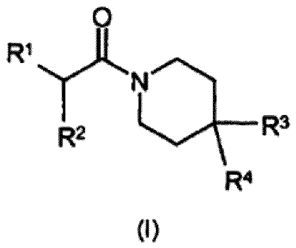 worin
R¹ und R² jeweils unabhängig voneinander aus gegebenenfalls substituiertem Aryl und gegebenenfalls substituiertem C₃₋₁₂-Alkyl ausgewählt sind; und
R³ und R⁴ jeweils unabhängig voneinander aus gegebenenfalls substituiertem C₁₋₆-Alkyl, gegebenenfalls substituiertem Phenyl und H ausgewählt sind.

2. Verwendung nach Anspruch 1, worin zumindest einer von R¹ und R² C₃₋₅-Alkyl ist.

3. Verwendung nach Anspruch 2, worin zumindest einer von R¹ und R² n-Butyl ist.

4. Verwendung nach einem der vorangegangenen Ansprüche, worin zumindest einer von R¹ und R² C₅₋₁₅-Aryl, vorzugsweise Phenyl, ist.

5. Verwendung nach Anspruch 4, worin (a) einer von R¹ und R² Phenyl und der andere n-Butyl ist oder (b) R¹ und R² gleich sind.

6. Verwendung nach Anspruch 5, worin (a) R¹ und R² beide n-Butyl sind oder (b) R¹ und R² beide Phenyl sind.

7. Verwendung nach einem der vorangegangenen Ansprüche, worin einer oder beide von R¹ und R² unsubstituiert sind.

8. Verwendung nach einem der vorangegangenen Ansprüche, worin (a) einer von R³ und R⁴ Hydroxyl und der andere H ist, (b) R³ und R⁴ beide H sind oder (c) einer oder beide von R³ und R⁴ substituiertes C₁₋₆-Alkyl ist/sind.

9. Verwendung nach Anspruch 8, worin einer oder beide von R³ und R⁴ Hydroxy-C₁₋₆-alkyl ist/sind.

10. Verwendung nach Anspruch 9, worin einer von R³ und R⁴ = -CH₂CH₂OH und der andere von R³ und R⁴ H ist.

11. Verwendung nach Anspruch 1, worin die Verbindung aus den Verbindungen 12.2, 12.1, 12.7, 12.4, 12.8, 12.9, 12.10, 12.11, 12.12, 4.1, 4.3, 9.2. 9.3 und 4.5 ausgewählt ist.

12. Verwendung nach einem der vorangegangenen Ansprüche, worin das Verfahren zur Prävention oder Reduktion von Fouling (a) ein Verfahren zur Prävention oder Reduktion von Mikrofouling oder Makrofouling; (b) ein Verfahren zur Reduktion oder Prävention von Biofilmbildung; (c) ein Verfahren zur Reduktion oder Prävention von Biofilmbildung durch eines oder mehrere von Bakterien, Pilzen, Algen und Einzeller; (d) ein Verfahren zur Reduktion oder Prävention von Biofilmbildung durch Bakterien; (e) ein Verfahren zur Reduktion oder Prävention von Makrofouling durch eines oder mehrere von Krustentieren, Moostierchen und Weichtieren; oder (f) ein Verfahren zur Reduktion oder Prävention von Makrofouling durch eines oder mehrere von Rankenfüßern und Muscheln sowie deren Larven; ist.

13. Verfahren zur Prävention oder Reduktion des Foulings eines Substrats, worin das Verfahren den Schritt des Aufbringens einer Verbindung wie in einem der Ansprüche 1 bis 11 definiert auf das Substrat umfasst.

14. Antifoulingzusammensetzung oder Überzugszusammensetzung, die eine Verbindung wie in einem der Ansprüche 1 bis 11 definiert umfasst.

## Revendications

1. Utilisation d'un composé de la formule (I) dans un procédé de réduction ou de prévention de salissures 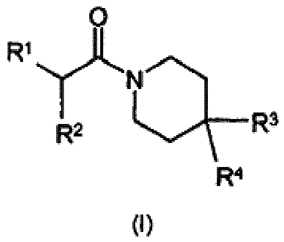 où
R¹ et R² sont indépendamment sélectionnés parmi aryle optionnellement substitué et alkyle C₃ à C₁₂ optionnellement substitué ; et
R³ et R⁴ sont indépendamment sélectionnés parmi hydroxyle, alkyle C₁ à C₆ optionnellement substitué, phényle optionnellement substitué et H.

2. Utilisation selon la revendication 1, où au moins un de R¹ et R² est alkyle C₃₋₅.

3. Utilisation selon la revendication 2, où au moins un de R¹ et de R² est n-butyle.

4. Utilisation selon l'une quelconque des revendications précédentes, où au moins un de R¹ et R² est aryle C₅ à C₁₅, de préférence phényle.

5. Utilisation selon la revendication 4, où (a) un de R¹ et R² est phényle et l'autre est n-butyle, ou (b) R¹ et R² sont les mêmes.

6. Utilisation selon la revendication 5, où (a) R¹ et R² sont tous les deux n-butyle, ou (b) R¹ et R² sont tous les deux phényle.

7. Utilisation selon l'une quelconque des revendications précédentes, où un ou les deux de R¹ et R² sont non substitués.

8. Utilisation selon l'une quelconque des revendications précédentes, où (a) un de R³ et R⁴ est hydroxyle et l'autre est H, (b) à la fois R³ et R⁴ sont H, ou ₍c₎ un ou les deux de R³ et R⁴ est alk^{y}le C₁ - C₆ substitué.

9. Utilisation selon la revendication 8, où un ou les deux de R³ et R⁴ est hydroxy alkyle C₁ - C₆.

10. Utilisation selon la revendication 9, où un de R³ et R⁴ est -CH₂CH₂₂OH et l'autre parmi R³ et R⁴ est H.

11. Utilisation selon la revendication 1, où le composé est sélectionné parmi les composés 12.2, 12.1, 12.7, 12.4, 12.8, 12.9, 12.10, 12.11, 12.12, 4.1, 4.3, 9.2, 9.3 et 4.5.

12. Utilisation selon l'une quelconque des revendications précédentes, dans laquelle le procédé de prévention ou de réduction des salissures est (a) un procédé de prévention ou de réduction de micro-salissures ou de macro-salissures ; (b) un procédé de réduction ou de prévention d'une formation de biofilm ; (c) un procédé de réduction ou de prévention d'une formation de biofilm par un ou plusieurs de bactéries, champignons, algues et protozoans ; (d) un procédé de réduction ou de prévention d'une formation de biofilm par des bactéries ; (e) un procédé de réduction ou de prévention de macro-salissures par un ou plusieurs de crustacés, bryozoans et mollusques ; ou (f) un procédé de réduction ou de prévention de macro-salissures par une ou plusieurs de barnacles et moules et leurs larves.

13. Procédé de prévention ou de réduction de salissures d'un substrat, où le procédé comprend l'étape consistant à appliquer au substrat un composé tel que défini dans l'une quelconque des revendications 1 à 11.

14. Composition anti-fouling ou composition de revêtement comprenant un composé tel que défini dans l'une quelconque des revendications 1 à 11.
